(19) Europäisches Patentamt
European Patent Office
Office européen des brevets

(11) **EP 2 412 404 A1**

(12) **EUROPEAN PATENT APPLICATION**
published in accordance with Art. 153(4) EPC

(43) Date of publication:
**01.02.2012 Bulletin 2012/05**

(51) Int Cl.:
**A61N 5/10** (2006.01)

(21) Application number: **09842200.9**

(22) Date of filing: **23.03.2009**

(86) International application number:
**PCT/JP2009/055702**

(87) International publication number:
**WO 2010/109586 (30.09.2010 Gazette 2010/39)**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicant: **National Institute of Radiological Sciences**
**Chiba-shi, Chiba 263-8555 (JP)**

(72) Inventors:
• **YAMAYA, Taiga**
  **Chiba-shi**
  **Chiba 263-8555 (JP)**

• **INANIWA, Taku**
  **Chiba-shi**
  **Chiba 263-8555 (JP)**
• **NISHIKIDO, Fumihiko**
  **Chiba-shi**
  **Chiba 263-8555 (JP)**
• **MURAYAMA, Hideo**
  **Chiba-shi**
  **Chiba 263-8555 (JP)**

(74) Representative: **Twelmeier Mommer & Partner Patent- und Rechtsanwälte**
**Westliche Karl-Friedrich-Strasse 56-68**
**75172 Pforzheim (DE)**

(54) **CONCEALMENT-TYPE RADIOTHERAPY/IMAGING COMBINED DEVICE**

(57)    A shielding structure against nuclear fragments (referred to as a shield) is operated according to ON and OFF of beam irradiation so as to reduce incidence of nuclear fragments on detectors during beam irradiation. This enables measurement of annihilation radiations and three-dimensional imaging of the irradiation field immediately after irradiation or even during irradiation.

Fig. 3

DURING IRRADIATION            AFTER IRRADIATION

**Description**

Technical Field

The present invention relates to a radiation therapy and imaging hybrid device that can reduce, when performing monitoring for detecting annihilation radiations occurring from an irradiation field due to radiation (also referred to as beam) irradiation in radiation therapy which is conducted by irradiating an affected area with X-rays or a particle beam, the incidence of nuclear fragments occurring from beam irradiation on detectors to enable measurement of annihilation radiations and three-dimensional imaging of the irradiation field immediately after irradiation or even during irradiation.

Background Art

Positron emission tomography (PET) is attracting attention as an effective test method for earlier diagnosis of cancer. In PET, a compound labeled with a trace amount of positron emitting nuclei is administered and annihilation radiations emitted from inside the body are detected to create an image of metabolic functions such as sugar metabolism and check for a disease and its extent. PET devices for practicing it have been put into actual use.

The principle of PET will be described below. A positron emitted from a positron emitting nuclide due to positron decay is annihilated with an ambient electron to produce a pair of 511-keV annihilation radiations, which are measured by a pair of radiation detectors based on the principle of coincidence counting. The position of the nuclide can thus be located on a single line (line of coincidence) that connects the pair of detectors. An axis from the patient's head to feet will be defined as a body axis. The distribution of nuclei on a plane that perpendicularly crosses the body axis is determined by two-dimensional image reconstruction from data on lines of coincidence on the plane, measured in various directions.

Early PET devices therefore have had a single-ring detector in which detectors are closely arranged in a ring shape on a plane to be the field of view so as to surround the field of view. With the advent of a multi-ring detector which includes a lot of single-ring detectors closely arranged in the direction of the body axis, the two-dimensional field of view has subsequently been extended to three dimensions. Since the 1990s, 3D mode PET devices have been actively developed which perform coincidence measurement even between detector rings with a significant improvement in sensitivity.

For cancer detected by the PET diagnosis or the like, treatments have a critical role. Approaches other than surgical operations and medication include radiation therapy of irradiating the affected area with radiations such as X-rays and gamma rays. In particular, particle radiotherapy of irradiating only a cancerous area with a heavy particle beam or proton beam is attracting much attention as a method both with an excellent treatment effect and a sharply concentrated irradiation characteristic with respect to the affected area. Among the methods of particle beam irradiation is conventional bolus irradiation where the irradiating beam is spread out to the shape of the affected area. In addition, spot scanning irradiation is under study, where the affected area is scanned with a pencil beam according to its shape etc. In any case, the directions and dosages of the irradiation beam are precisely controlled according to a treatment plan that is thoroughly calculated based on X-ray CT images or the like obtained separately.

The patient positioning accuracy is the key to administer treatment exactly according to the treatment plan. The irradiation field is often positioned based on X-ray images. In general, X-ray images fail to provide a sufficient contrast between tumor and normal tissues, and it is difficult to identify a tumor itself for positioning. In addition to such misalignment of the irradiation field at the time of patient setup, other problems have been pointed out such as a change in the size of the tumor from the time of creation of the treatment plan, and respiratory and other variations of the tumor position. Under the present circumstances, it is difficult to accurately identify whether irradiation is performed according to the treatment plan. Even if the actual irradiation field deviates from the treatment plan, it is not easy to detect.

To solve the foregoing problems, attention is being given to a method of imaging the irradiation field in real time using PET techniques. In the method, no PET medicine is administered. Instead, annihilation radiations caused by particle beam irradiation or X-ray irradiation through a projectile fragmentation reaction, target fragmentation reaction, and photonuclear reaction are rendered into an image by using the principle of PET. Therapy monitoring is said to be possible since the positions of occurrence of annihilation radiations has a strong correlation with the dose distribution of the irradiation beam. (W. Enghardt, et al. "Charged hadron tumour therapy monitoring by means of PET," Nucl. Instrum. Methods A 525, pp. 284-288, 2004. S. Janek, et al. "Development of dose delivery verification by PET imaging of photonuclear reactions following high energy photon therapy," Phys. Med. Biol., vol. 51 (2006) pp. 5769-5783). In heavy particle radiotherapy, direct irradiation of positron-emitting nuclei such as $^{11}C$, instead of ordinary stable nuclei such as $^{12}C$, can eliminate a mismatch between the positions of occurrence of annihilation radiations and the dose distribution, as well as improve the S/N ratio of PET images.

Device requirements for PET that images an irradiation field in real time (hereinafter, referred to as beam on-line PET) are summarized into the following four points:

1. The detectors not obstruct the treatment beam.
2. The detectors not drop in performance due to nuclear fragments (charged particles and/or neutrons generated by collision of incident particles and target nuclei).
3. PET measurement can be performed immediately after irradiation or even during irradiation for efficient measurement of short life RIs, in order to enhance the precision of PET images and shorten the patient binding time.
4. The irradiation field can be imaged in a three-dimensional fashion.

Concerning the foregoing requirement 2, the incident of nuclear fragments on detectors can radioactivate the scintillators themselves that constitute the detectors. This may lead to the omission of annihilation radiations to be measured and the production of errors in position information. In heavy particle beam irradiation, both charged particles and neutrons occur as nuclear fragments. In proton beam irradiation, neutrons are considered to be dominant. In either case, nuclear fragments are generated with forward directivity with respect to the treatment beam. It has been reported that the forward directivity is accompanied by a wide angle. (N. Matsufuji, et al., "Spatial fragmentation distribution from a therapeutic pencil-like carbon beam in water," Physics in Medicine and Biology 50(2005) 3393-3403, S. Yonai, et al., "Measurement of neutron ambient dose equivalent in passive carbon-ion and proton radiotherapies," Medical Physics 35 (2008) 4782-4792).

As for the requirement 3, the nuclei generated by the radiation irradiation have an extremely short half-life of several tens of seconds to 20 minutes or so. The nuclei can also move inside the living body due to blood flow and other factors. Immediate PET measurement during irradiation is thus desired.

The GSI Laboratory in Germany and the National Cancer Center Hospital East in Japan are making attempts for beam on-line PET by using an opposed gamma camera type PET device in which two PET detectors of flat type are arranged with the bed of a treatment device therebetween. (P. Crespo, et al., "On the detector arrangement for in-beam PET for hadron therapy monitoring," Phys. Med. Biol., vol. 51 (2006) pp. 2143-2163, T. Nishio, et al., "Dose-volume delivery guided proton therapy using beam ON-LINE PET system," Med. Phys., vol. 33 (2006) pp. 4190-4197). The opposed gamma camera type device satisfies the requirements 1, 2, and 3 since the detectors can be arranged away from the beam path. However, lines of coincidence measured are highly uneven in direction, some information necessary for image reconstruction missing. This significantly reduces the resolution in directions perpendicular to the detector plane, failing to meet the requirement 4.

There have also been proposed methods of implementing opposed gamma camera type PET device on a rotating treatment gantry in which the treatment beam irradiation device itself rotates around a patient (Japanese Patent Application Laid-Open No. 2008-22994, Japanese Patent Application Laid-Open No. 2008-173299). The requirement 3 is not satisfied, however, since the opposed gamma camera type PET device can be rotated only after beam irradiation, except in some rare cases where beam irradiation is continuously performed from various directions.

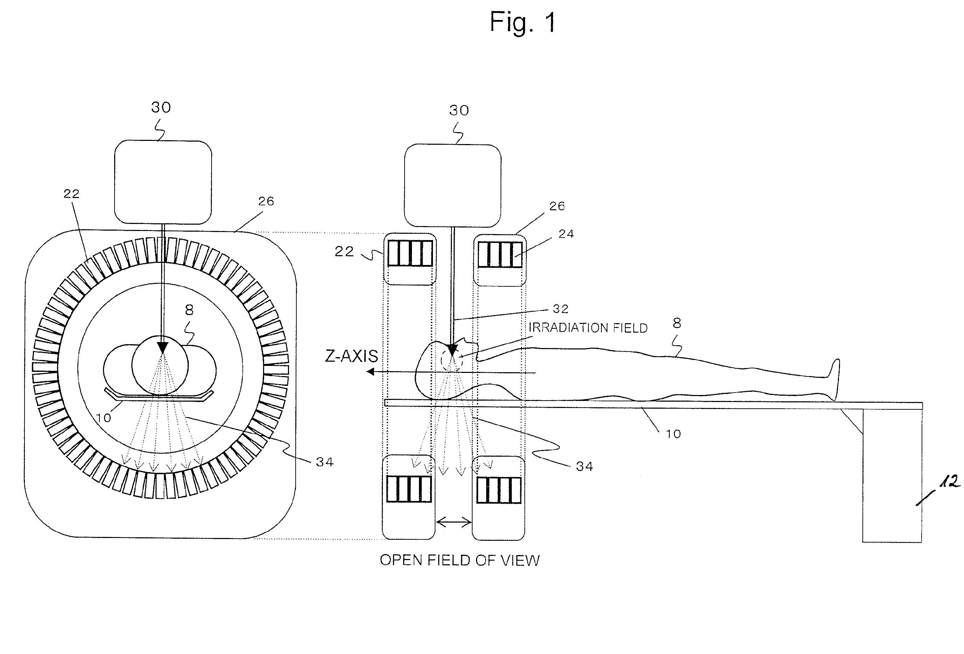

The applicant has proposed an open PET device as a method that provides a gap for allowing the passage of a treatment beam and is capable of three-dimensional imaging without rotation of the PET device. In the open PET device, as shown in Fig. 1, two separate multi-ring detectors 22 and 24 are arranged apart in the direction of the body axis of a patient 8 to form a physically open field of view area (also referred to as an open field of view) (Taiga Yamaya, Taku Inaniwa, Shinichi Minohara, Eiji Yoshida, Naoko Inadama, Fumihiko Nishikido, Kengo Shibuya, Chih Fung Lam and Hideo Murayama, "A proposal of an open PET geometry," Phy. Med. Biol., 53, pp. 757-773, 2008.). Images in the open field of view are reconstructed from lines of coincides between the two separate detector rings 22 and 24. In the diagram, 10 designates a bed, 12 designates a pedestal of the bed, 26 designates a gantry cover, and 30 denotes an irradiation port. Such an open PET device meets the requirements 1, 3, and 4. Without an open field of view of sufficient width, nuclear fragments 34 caused by a treatment beam 32 that enters the open field of view from the irradiation port 30 may be incident on detectors on both sides of the open field of view. If the treatment intensity is extremely high, the detectors may get radioactivated and fail to meet the requirement 2.

Disclosure of the Invention

The present invention has been achieved in order to solve the foregoing conventional problems. It is an object of the present invention to reduce the incidence of nuclear fragments accompanying beam irradiation on detectors to enable measurement of annihilation radiations and three-dimensional imaging of the irradiation field immediately after irradiation or even during irradiation.

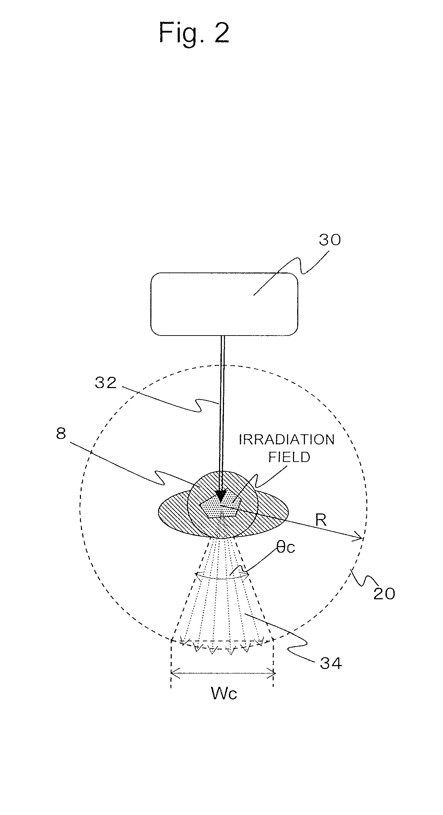

Fig. 2 shows a situation in which nuclear fragments 34 are incident on detectors. Wc represents the range (hereinafter, referred to as a critical region) where the number of nuclear fragment particles 34 incident on detectors exceeds an allowable value. The relationship between the angle of view $\theta_c$ from the center of a detector ring 20 and Wc is expressed by:

$$\theta_C = 2\sin^{-1}(W_C / (2R)).$$

Here, R is the radius of the detector ring 20. While Fig. 2 shows a plane perpendicular to the body axis, nuclear fragments also scatter in the direction of the body axis (referred to as the direction of the Z-axis) with the angle of view $\theta_C$.

Then, the present invention is directed to operating a shielding structure against nuclear fragments (referred to as a shield) according to the ON and OFF of beam irradiation so as to reduce the incidence of nuclear fragments on detectors during beam irradiation.

The requirements for the shield are as follows:

1. In order to perform measurement for imaging during irradiation, the coverage of the shield shall be minimized so that at least unshielded detectors can be used for measurement for imaging.

2. The material and thickness of the shield are desirably optimized to suppress the radioactivation of the detectors to or below an allowable value while allowing any transmission of annihilation radiations to be measured.

3. The material and thickness of the shield may be uniform, but are preferably designed so that the shielding effect decreases as the scattering angle increases, considering the forward scattering of nuclear fragments.

4. In order to move the shield at high speed and simplify the mechanism, it is important that the shield have a light weight. The foregoing optimization in the size and material of the shield also allows a weight reduction of the shield.

The present invention has been achieved based on the foregoing findings, and attains the foregoing object by the provision of a radiation therapy and imaging hybrid device including an imaging device that has a detector arranged so as to be able to measure a radiation occurring from an affected area due to radiation irradiation and images an irradiation field after irradiation or during irradiation in synchronization with a radiation with which a field of view of the detector is irradiated, the hybrid device including: a radiation therapy device that irradiates a part of a subject with a radiation, the part lying in a field of view of the imaging device; and shielding means for lessening incidence of nuclear fragments on the detector, the nuclear fragments flying forward in a direction of irradiation from the subject due to the radiation irradiation, the shielding means being arranged in a flying space of the nuclear fragments.

The present invention also attains the foregoing object by the provision of a radiation therapy and imaging hybrid device including a PET device that has a group of detectors arranged around a subject so as to be capable of coincidence measurement of a pair of annihilation radiations occurring from the subject and performs tomographic scanning on the subject after irradiation or during irradiation in synchronization with a radiation with which a field of view of the group of detectors is irradiated, the hybrid device including: a radiation therapy device that irradiates a part of the subject with a radiation, the part lying in a field of view of the PET device; and shielding means for lessening incidence of nuclear fragments on the detectors, the nuclear fragments flying forward in a direction of irradiation from the subject due to the radiation irradiation, the shielding means being arranged in a flying space of the nuclear fragments.

Here, the group of detectors may be arranged in a ring shape around an axis of the subject. The shielding means may include a shield that can be inserted and retracted into/from the flying space and lessens passage of nuclear fragments, and control means for controlling the insertion and retraction of the shield. The shield may be located in the flying space during radiation irradiation to lessen incidence of fragments into the detectors.

The shield may make a rotational movement.

The shield may make a reciprocal movement.

The shield may be immediately retracted after radiation irradiation.

The shield may be rotated depending on a position of an irradiation port.

The shield and a drive unit thereof may be integrated with a bed.

A material or thickness of the shield may be changed according to a scattering angle of the nuclear fragments.

The present invention also provides a control program of a detector rotation type radiation therapy and imaging hybrid device including an imaging device that has a detector arranged so as to be able to measure a radiation occurring from an affected area due to radiation irradiation and images an irradiation field after irradiation or during irradiation in synchronization with a radiation with which a field of view of the detector is irradiated, the control program controlling shielding means so as to lessen incidence of nuclear fragments on the detector, the nuclear fragments flying forward in a direction of irradiation from the subject due to the radiation irradiation, the shielding means being arranged in a flying space of the nuclear fragments.

The present invention also provides a control program of a detector rotation type radiation therapy and imaging hybrid device including a PET device that has a group of detectors arranged around a subject so as to be capable of coincidence measurement of a pair of annihilation radiations occurring from the subject and performs tomographic scanning on the subject after irradiation or during irradiation in synchronization with a radiation with which a field of view of the group of detectors is irradiated, the control program controlling shielding means so as to lessen incidence of nuclear fragments on the detectors, the nuclear fragments flying forward in a direction of irradiation from the subject due to the radiation irradiation, the shielding means being arranged in a flying space of the nuclear fragments.

According to the present invention, when performing monitoring for detecting annihilation radiations occurring from an irradiation field due to radiation irradiation in radiation therapy which is conducted by irradiating an affected area with X-rays or a particle beam, the incidence of nuclear fragments accompanying the beam irradiation on detectors can be reduced to enable measurement of annihilation radiations and three-dimensional imaging of the irradiation field immediately after irradiation or even during irradiation.

Brief Description of the Drawings

Fig. 1 includes a front view and a side view showing an open PET device proposed by the applicant;
Fig. 2 is a side view showing a conventional problem;
Fig. 3 includes side views and front views showing a first embodiment of the present invention;
Fig. 4 is a block diagram showing a configuration for synchronizing beam irradiation with the insertion and retraction of shields according to the first embodiment;
Fig. 5 is a flowchart showing a typical procedure of a shield operation in synchronization with beam irradiation according to the present invention;
Fig. 6 is a time chart showing how the ON and OFF of beam irradiation and the shield operation are synchronized in the first embodiment;
Fig. 7 is a side view showing a second embodiment of the present invention;
Fig. 8 is a block diagram showing a configuration for synchronizing beam irradiation with the insertion and retraction of shields according to the second embodiment;
Fig. 9 is a flowchart showing the procedure of an example of the second embodiment;
Fig. 10 is a diagram showing the procedure of another example of the second embodiment;
Fig. 11 is a time chart showing how the ON and OFF of beam irradiation and the shield operation are synchronized in the second embodiment;
Fig. 12 is a side view showing a third embodiment of the present invention;
Fig. 13 is a time chart showing how the ON and OFF of beam irradiation and the shield operation are synchronized in the third embodiment;
Fig. 14 is a plan view showing an example in which a PET detector ring is obliquely arranged;
Fig. 15 includes a side view and a front view showing an example with horizontal and vertical, two irradiation ports;
Fig. 16 is a diagram showing the operation of the same;
Fig. 17 includes a side view and a front view showing another example with horizontal and vertical, two irradiation ports;
Fig. 18 is a diagram showing the operation of the same;
Fig. 19 includes a side view and a front view showing an example with a rotating irradiation gantry;
Fig. 20 is a diagram showing the operation of the same;
Fig. 21 includes a side view and a front view showing an example in which shields are built in a bed;
Fig. 22 is a diagram showing the operation of the same;
Fig. 23 includes a side view and a front view showing an example in which arc-shaped end shields are installed as shields;
Fig. 24 is a diagram showing the operation of the same;
Fig. 25 includes a side view and a front view showing an example of shield rotation type; and
Fig. 26 includes a side view and a front view showing another example of the same.

Best Mode for Carrying Out the Invention

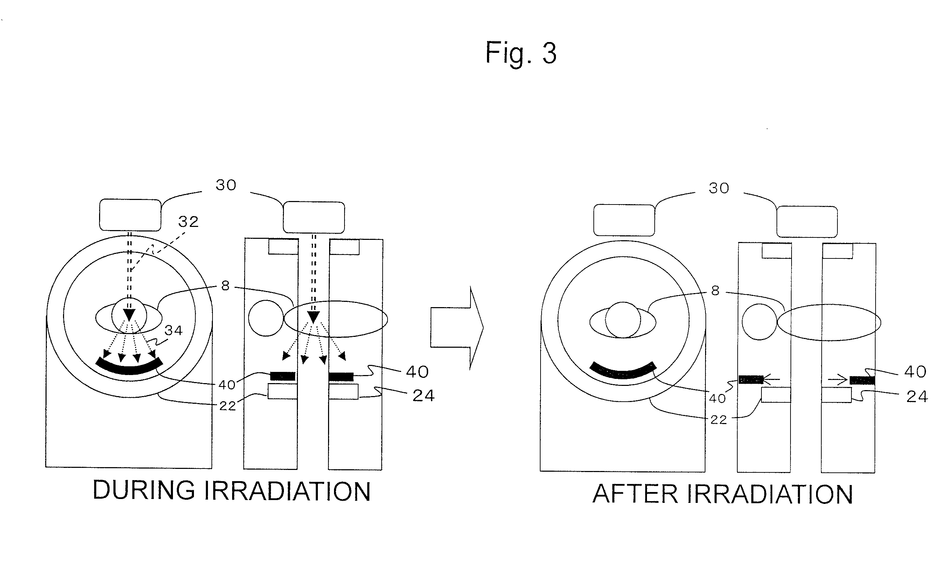

Hereinafter, embodiments of the present invention will be described in detail with reference to the drawings.
As shown in Fig. 3, a first embodiment of the present invention is a typical example of shield retraction type, in which shields 40 are retracted immediately after irradiation so as to allow high-sensitivity PET measurement with all detectors. As will be illustrated in examples to be described later, the shields 40 have various patterns of shape and retracting method. While Fig. 3 shows an example of perpendicular single field irradiation, the present embodiment may be applied to horizontal and vertical double field irradiation as well as a rotating gantry irradiation system with a rotating irradiation port.

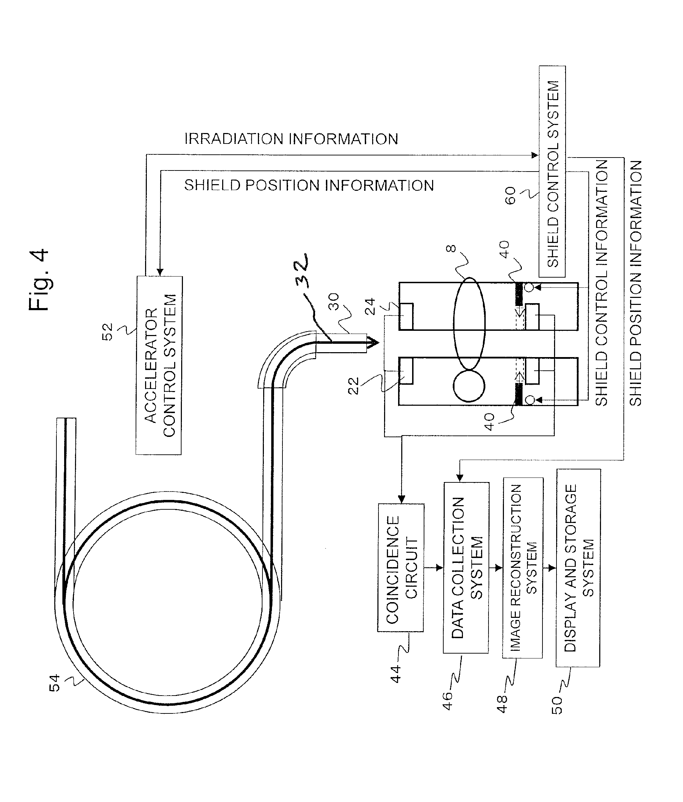

Fig. 4 shows a mechanism for synchronizing beam irradiation with the insertion and retraction of the shields 40. The irradiation timing of a treatment beam 32 is controlled by an accelerator control system 52. A shield control system 60 performs control for inserting and retracting the shields 40 according to irradiation information received from the accelerator control system 52. The shield control system 60 also has the function of transmitting current position information on the shields 40 to the accelerator control system 52. In the diagram, 54 designates a synchrotron.

Single event data on annihilation radiations detected by detectors such as PET detectors is converted by a coincidence circuit 44 into coincidence data for identifying lines of coincidence. The coincidence data is stored into a data collection system 46 in succession. After accumulation of measurement data for a certain period of time, an image reconstruction system 48 performs an image reconstruction operation, whereby images of the irradiation field are displayed or stored on/in a display and storage system 50. The time width for accumulating measurement data will be referred to as a time frame. The processing systems of the PET measurement data may basically continue processing and collecting measurement data independent of the accelerator control system 52 and the shield control system 60. It is needed, however, to include a shield position signal or the like into the coincidence data so that data selection and sensitivity correction can be properly performed at the time of image reconstruction.

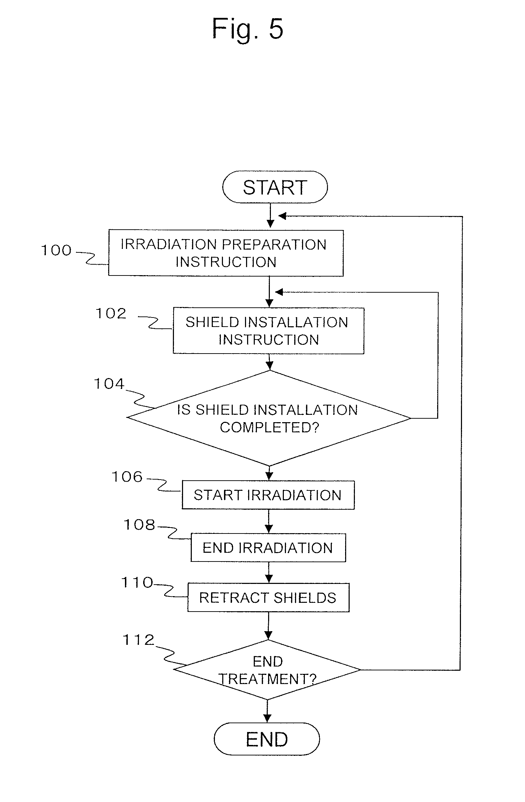

Fig. 5 shows a typical procedure of shield operation in synchronization with beam irradiation. The shield control system 60 acquires an irradiation preparation instruction (step 100), and moves the shields 40 to predetermined shielding positions (step 102). The accelerator control system 52 acquires information on the completion of shield installation (step 104), and starts irradiation (step 106). The shield control system 60 acquires information on the end of irradiation (step 108), and immediately retracts the shields 40 (step 110). To perform several separate irradiations, the foregoing shield installation → irradiation → shield retraction are repeated until a series of irradiations are completed to end the treatment (step 112).

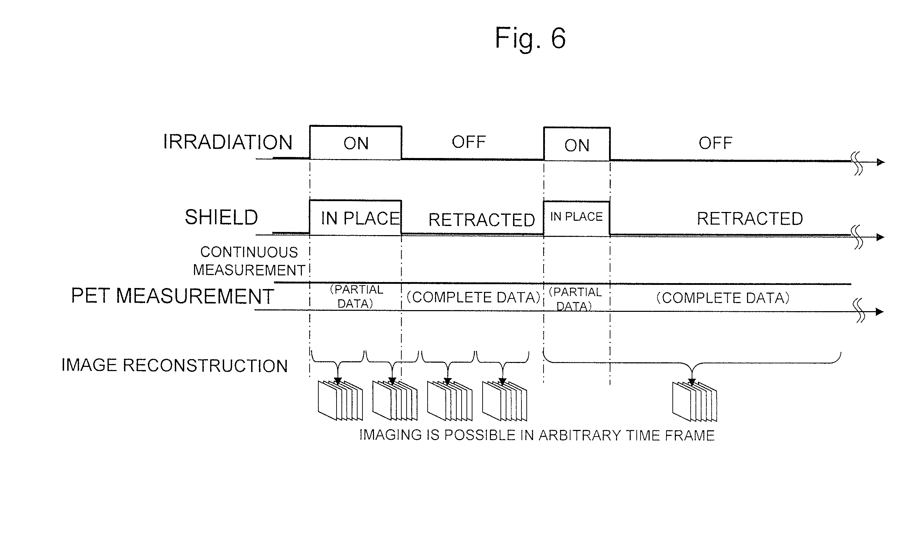

Fig. 6 shows how the ON and OFF of the irradiation and the shield operation are synchronized. The shields need to be located in the predetermined positions while irradiation is ON. PET measurement itself may be continued independent of the irradiation and the shield operation. During irradiation, i.e., when the shields cover some of the detectors, the sensitivity of the detectors drops significantly. The measurement data during irradiation is thus partial data with limited lines of coincidence. It is therefore desired that the end of irradiation be followed by the retraction of the shields as soon as possible so that a lot of complete data is obtained without missing lines of coincidence.

For PET measurement, the collection of coincidence data is continued. Data corresponding to a time frame specified afterward is extracted for image reconstruction. Alternatively, a time frame may be specified in advance, and PET measurement may be performed only for the time frame specified. If an image degradation results from missing lines of coincidence or a decrease in the number of counts of annihilation radiations measured, a longer time frame may be set to improve the S/N ratio of the measurement data. It is known that prompt gamma rays are also emitted from the irradiation field during irradiation aside from annihilation radiations. Prompt gamma rays can increase random coincidence which is a noise component to PET measurement. In view of ON/OFF periodicity of microsecond order even during irradiation, countermeasures have been proposed that use only measurement data in OFF states for image reconstruction, excluding measurement data in ON states. (P. Crespo, et al., "Suppression of random coincidences during in-beam PET measurements at ion beam radiation therapy facilities," IEEE TRANSACTIONS ON NUCLEAR SCIENCE, VOL. 52, NO. 4, AUGUST 2005). Alternatively, imaging may be performed only immediately after irradiation, not during irradiation.

Among methods of particle beam irradiation is conventional bolus irradiation where the irradiating beam is spread out to the shape of the affected area. Spot scanning irradiation is also under study, where the affected area is scanned with a pencil beam according to its shape etc. The present invention is applicable to both the irradiation methods. A synchrotron basically makes intermittent operations to repeat beam irradiation ON and OFF periodically. The shields may thus be inserted and retracted in synchronization with the irradiation periods. The shields may be retracted after the end of a series of irradiations. The latter case also corresponds to when a beam is continuously produced from a synchrotron, instead of periodical irradiations planned for spot scanning irradiation.

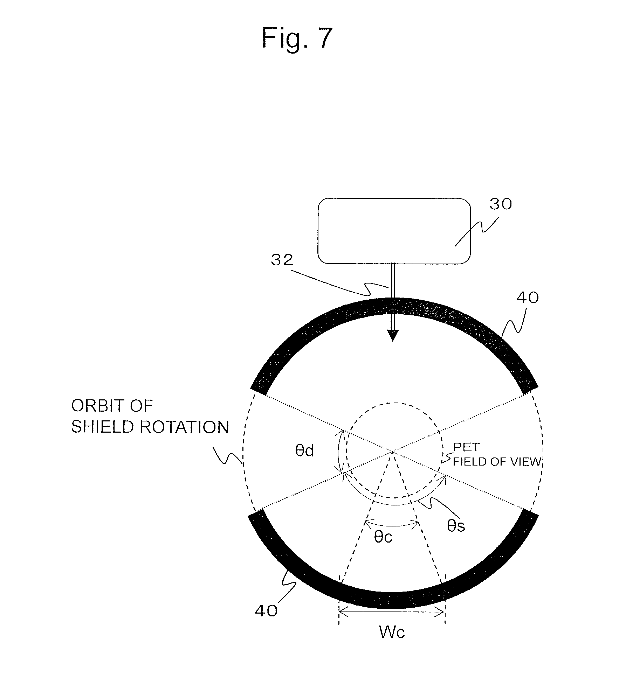

Fig. 7 shows a second embodiment (shield rotation type) in which arc-shaped shields 40 are rotated along the detector rings instead of the shields being retracted. Such a method is limited to periodical irradiation of repeating beam irradiation ON and OFF periodically. The beam irradiation and the shield rotation can be synchronized to reduce the incidence of nuclear fragments on detectors. The shield rotation type can reduce a mechanical burden as compared to the shield retraction type when the shields are inserted and retracted in synchronization with short irradiation cycles.

Specifically, a pair of shields are opposed to each other with respect to the Z-axis, the body axis of the patient. There is provided a mechanism for rotating the shields about the Z-axis independent of the irradiation port 30. There are two pairs of shields 40 since there are two detector rings arranged in the Z direction with the open space therebetween. The two pairs of shields 40 rotate in the same rotation period and phase. Assuming that the angle of view of each shield 40 as seen from the Z-axis is $\theta s$, the angle of view $\theta d$ of detectors that are not covered with the shields is expressed by $\theta d = 180° - \theta s$.

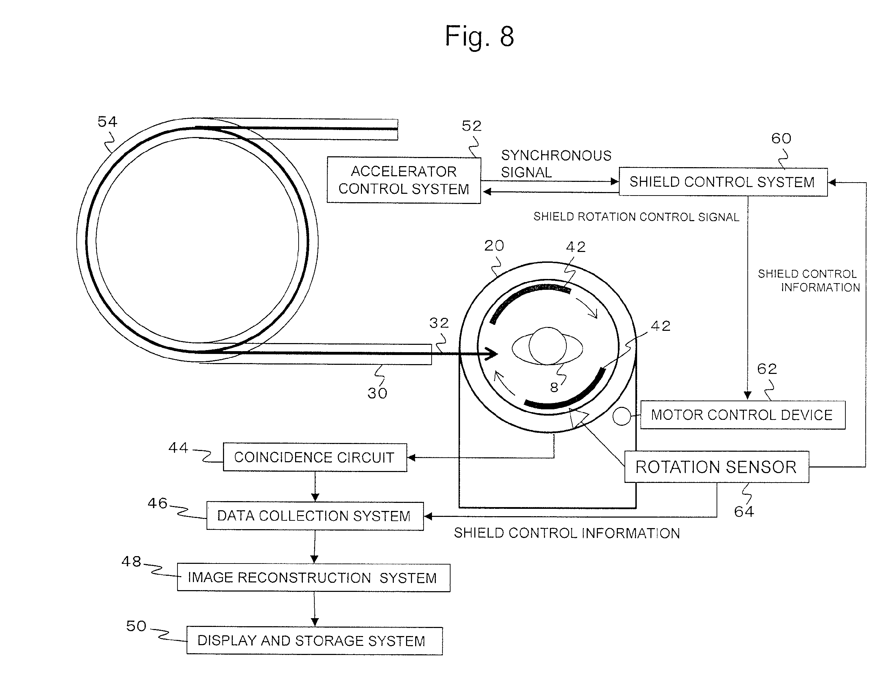

Fig. 8 shows a mechanism for synchronizing beam irradiation with the rotation of the shields 40. The irradiation period of the treatment beam 32 is controlled by the accelerator control system 52. The shield control system 60 transmits a rotation control signal to a motor control device 62 so that the shield rotation is in synchronization with a synchronous signal received from the accelerator control system 52. Information on the position and rotation speed of the shields 40 is successively transmitted from a rotation sensor 64 to the shield control system 60. The data flow for PET measurement is the same as in Fig. 4.

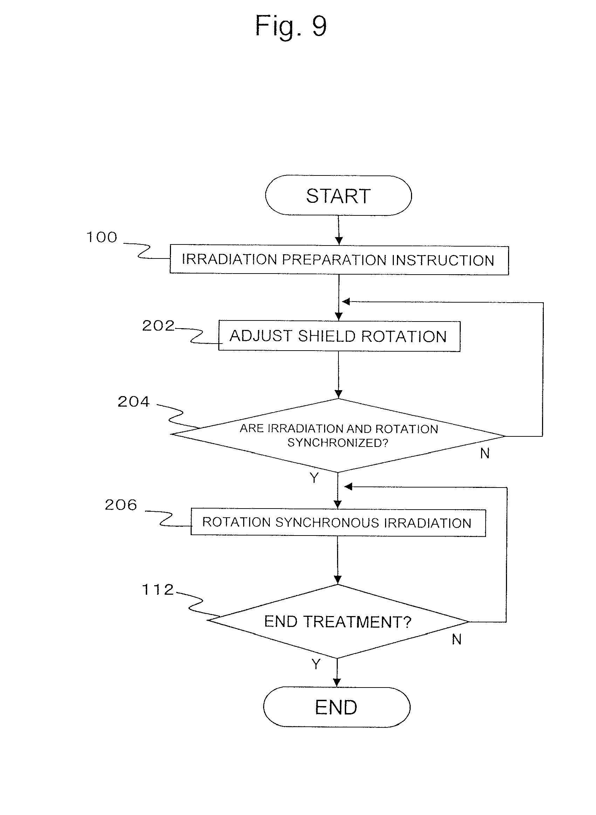

Fig. 9 shows a typical procedure for beam irradiation in synchronization with shield rotation. The shield control system 60 acquires an irradiation preparation instruction (step 100), and makes adjustments so as to synchronize the operation period of the synchrotron with the shield rotation (step 202). If the irradiation and the shield rotation are synchronized (step 204), rotation synchronous irradiation (step 206) is repeated to perform irradiation only when all detectors lying in a critical region are covered with the shields 40.

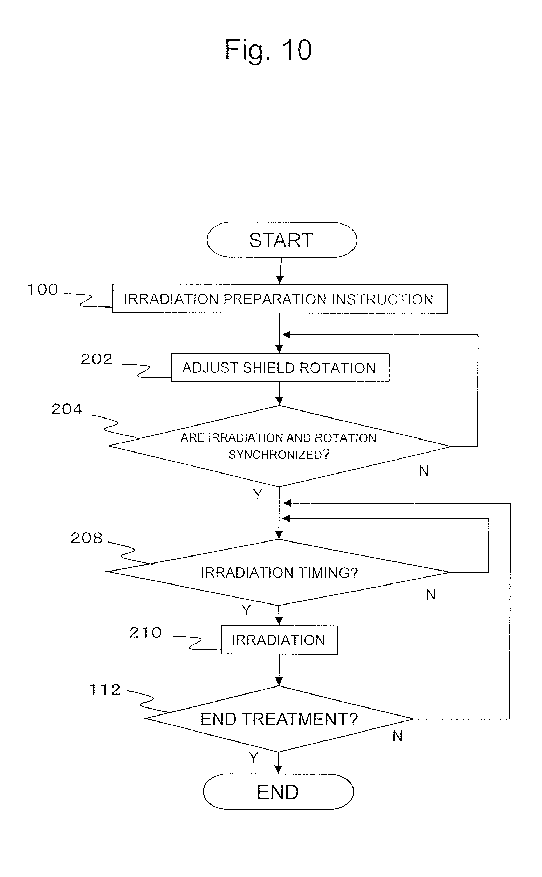

Fig. 9 shows the procedure in which the accelerator control system 52 controls the rotation synchronous irradiation on the assumption that the irradiation and rotation are stably in synchronization. When, for example, the shield rotation is unstable, as shown in Fig. 10, the shield control system 60 may check the shield positions and transmit irradiation timing information to the accelerator control system 52 (step 208) for irradiation (step 210).

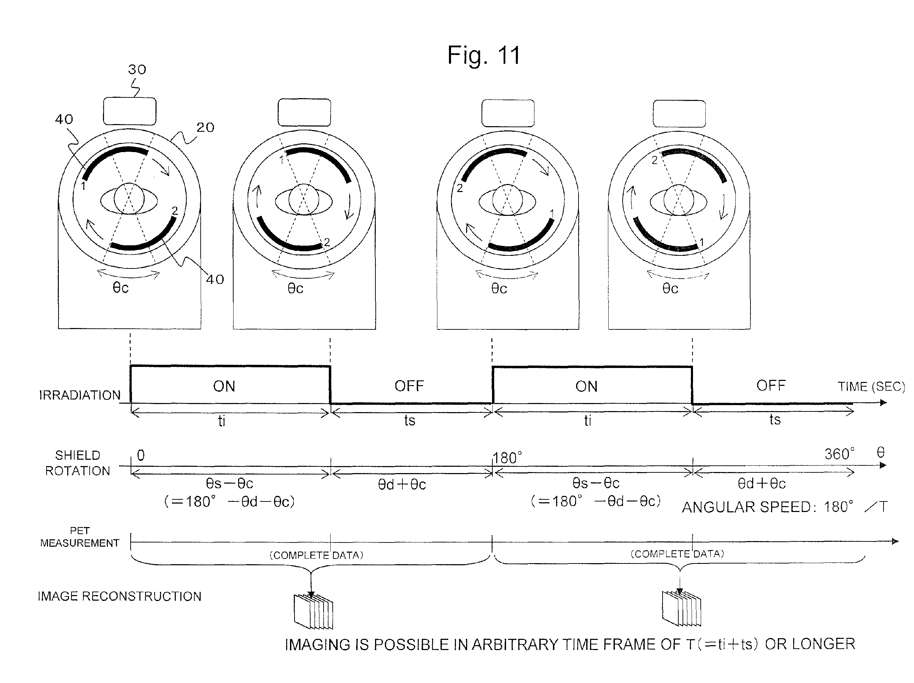

Fig. 11 shows how beam irradiation and shield rotation are synchronized to reduce the incidence of nuclear fragments on detectors. The irradiation of the treatment beam is performed only when all the detectors lying in the critical region are covered with the shields. The treatment beam is operated in periods of $T = ti + ts$ sec, with $ti$ sec of irradiation and $ts$ sec on standby. The shields 40 make a single rotation in $2T$ sec. The condition on $\theta d$, a parameter related to shield size, will now be described below. The lower limit of $\theta d$ is:

$$\theta d \geq 2\sin^{-1}(r \ / \ R'),$$

where R' is the radius of the orbit of the shields, and r is the radius of the PET field of view. The upper limit of $\theta d$ is:

$$\theta d \leq ts \ / \ T \times 180° - \theta c.$$

Here, $\theta s = 180° - \theta d$. Since the shields always cover detectors irrespective of the ON or OFF of irradiation, the shield rotation type can only acquire limited lines of coincidence all the time. Lines of coincidence from various angles are needed for image reconstruction. The minimum value of the time frame capable of imaging is thus an irradiation clock of T sec which is equivalent to a 180° rotation of the shields.

The Heavy Ion Medical Accelerator in Chiba (HIMAC) of the National Institute of Radiological Sciences in Japan controls treatment beams in periods of $T = 3.3$ sec. The present invention will be described here in terms of application to HIMAC. Assuming that the radius of the orbit of the shields R' = 50 cm and the radius of the PET field of view r = 20 cm, the lower limit of $\theta d$ is $\theta d \geq 47.2°$. Table 1 shows upper limits of $\theta d$ for different irradiation durations ti and different widths Wc of the critical region. ts = 3.3 - ti. No device is feasible if the upper limit falls below the lower limit (in the table, denoted as NA). For enhanced sensitivity of the PET device, it is actually desirable to employ the maximum $\theta d$ (i.e., the minimum $\theta s$).

[Table 1]

|  | Wc=20cm | Wc=30cm | Wc=40cm | Wc=50cm | Wc=60cm |
|---|---|---|---|---|---|
| ti=0.5 sec | 129.7° | 117.8° | 105.6° | 92.7° | 79.0° |
| ti=1.0 sec | 102.4° | 90.5° | 78.3° | 65.5° | 51.7° |
| ti=1.5 sec | 75.1° | 63.3° | 51.0° | NA | NA |
| ti=2.0 sec | 47.8° | NA | NA | NA | NA |

While the present example has dealt with the case with a single irradiation port, the present invention is also applicable to a case with a plurality of irradiation ports such as vertical and horizontal ones. Typically, a plurality of irradiation ports are not simultaneously used for treatment beam irradiation. The phase of shield rotation or the phase of beam irradiation may therefore be relatively changed depending on the movement of the port for beam irradiation. The same holds for a rotating irradiation gantry.

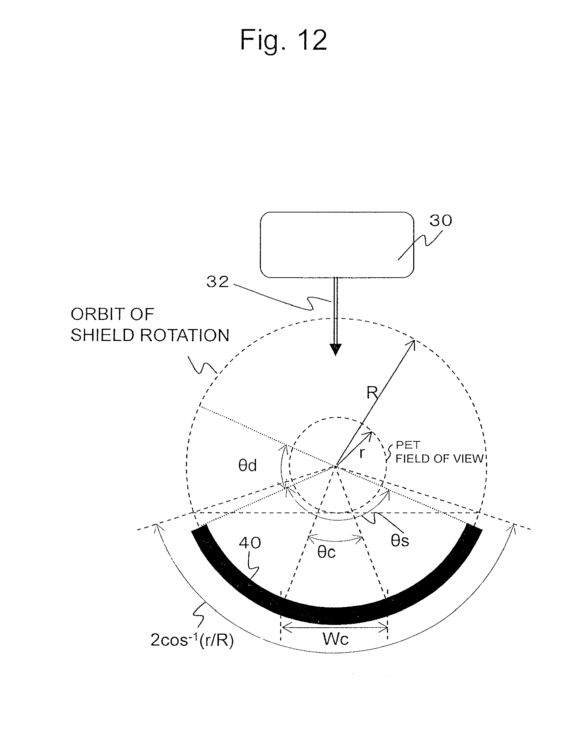

Fig. 12 shows a third embodiment of shield rotation type, in which a single shield, not a pair, is rotated. Two detector rings arranged in the direction of the Z-axis with an open space therebetween include respective shields 40 which are controlled with the same rotation period and phase. Again, the shields 40 have an angle of view of θs, whereas the angle of view of detectors not covered with the shields 40 is θd + 180°.

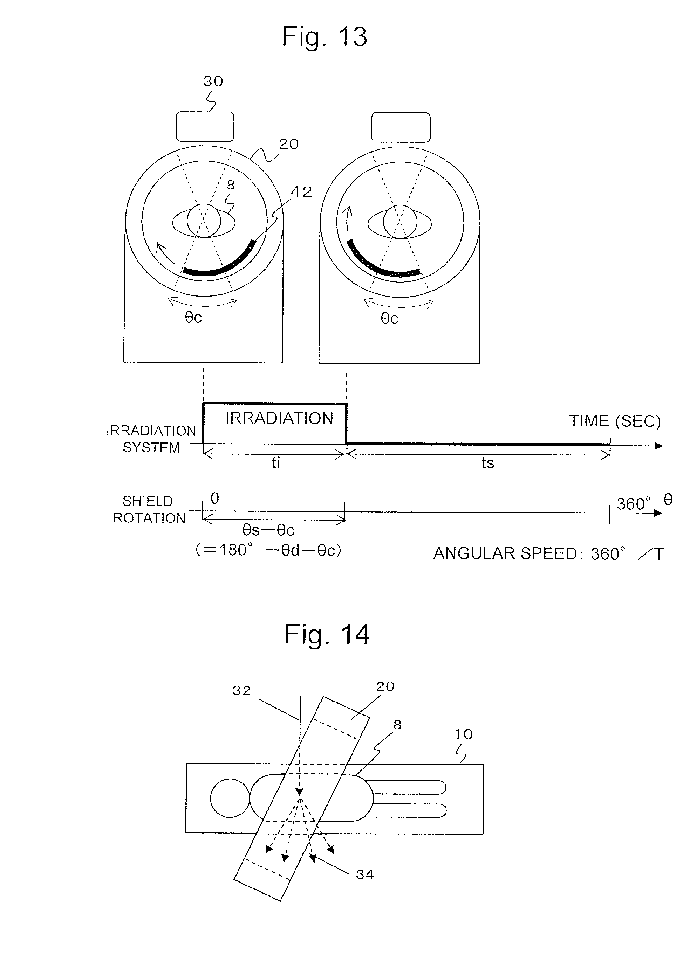

Fig. 13 shows how beam irradiation and shield rotation are synchronized to reduce the incidence of nuclear fragments on detectors. Again, the treatment beam is operated in periods of T = ti + ts sec, with ti sec of irradiation and ts sec on standby. The irradiation of the treatment beam is performed only when all the detectors lying in the critical region are covered with the shields. The shields need to make a single rotation in T sec. The upper limit of θs is:

$$\theta s \leq 2\cos^{-1}(r \; / \; R'),$$

where R' is the radius of the orbit of the shields, and r is the radius of the PET field of view. The lower limit of θs is:

$$\theta s \geq ti \; / \; T \times 360° + \theta c.$$

θs will also be examined in terms of application to HIMAC. Assuming that the radius of the orbit of the shields R' = 50 cm and the radius of the PET field of view r = 20 cm, the upper limit of θs is θs ≤ 132.8°. Table 2 shows lower limits of θs for different irradiation durations ti and different widths Wc of the critical region. ts = 3.3 - ti. No device is feasible if the upper limit falls below the lower limit (in the table, denoted as NA). For enhanced sensitivity of the PET device, it is actually desirable to employ the minimum θs. As compared to the case of Table 1, given the same beam irradiation period T, shorter irradiation durations ti are needed since the shield rotation speed is twice as high.

[Table 2]

|            | Wc=20cm | Wc=30cm | Wc=40cm | Wc=50cm | Wc=60cm |
|------------|---------|---------|---------|---------|---------|
| ti=0.5 sec | 77.6°   | 89.5°   | 101.7°  | 114.5°  | 128.3°  |
| ti=1.0 sec | 132.2°  | NA      | NA      | NA      | NA      |
| ti=1.5 sec | NA      | NA      | NA      | NA      | NA      |
| ti=2.0 sec | NA      | NA      | NA      | NA      | NA      |

The present invention (shield retraction type and shield rotation type) may be applied to devices other than open PET devices. Fig. 14 is a prior example in which an ordinary PET device is obliquely arranged. (P. Crespo, et al., "On the detector arrangement for in-beam PET for hadron therapy monitoring," Phys. Med. Biol., vol. 51 (2006) pp. 2143-2163). There is secured a beam irradiation path, whereas nuclear fragments 34 can be incident on detectors as shown in the diagram. Here, the incidence of nuclear fragments on the detectors can be reduced by installing a shield in the position where nuclear fragments are incident, at least during irradiation. The shield may be retracted in response to the ON and OFF of the beam. The shield may be rotated inside the detector ring if the ON and OFF of the beam are periodically repeated.

The imaging device need not necessarily be a PET device, and may be a SPECT device with a gamma camera etc. In such a case, it would be possible to measure prompt gamma rays as a signal aside from annihilation radiations.

[Examples]

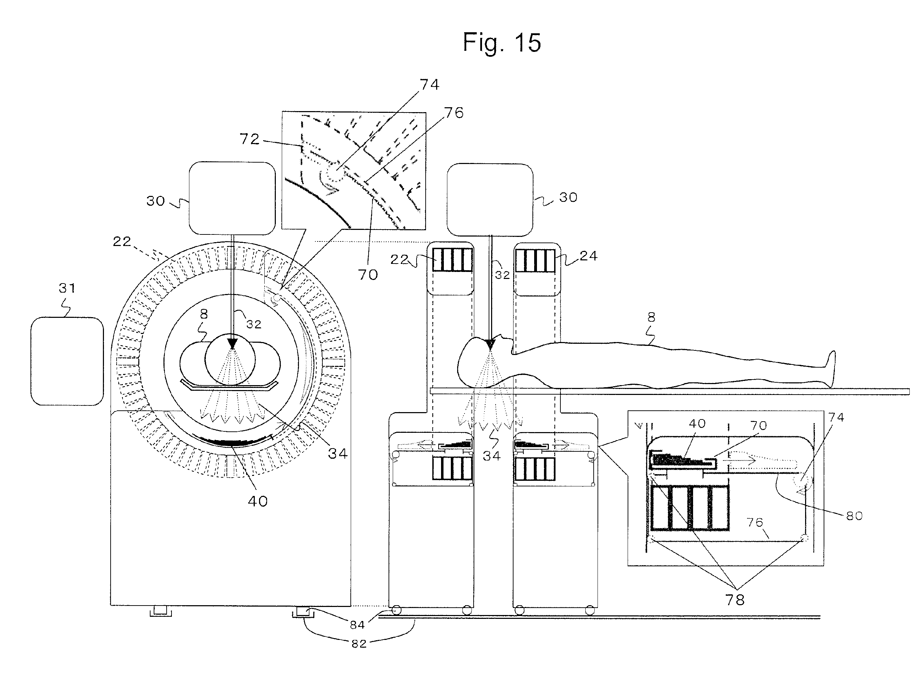

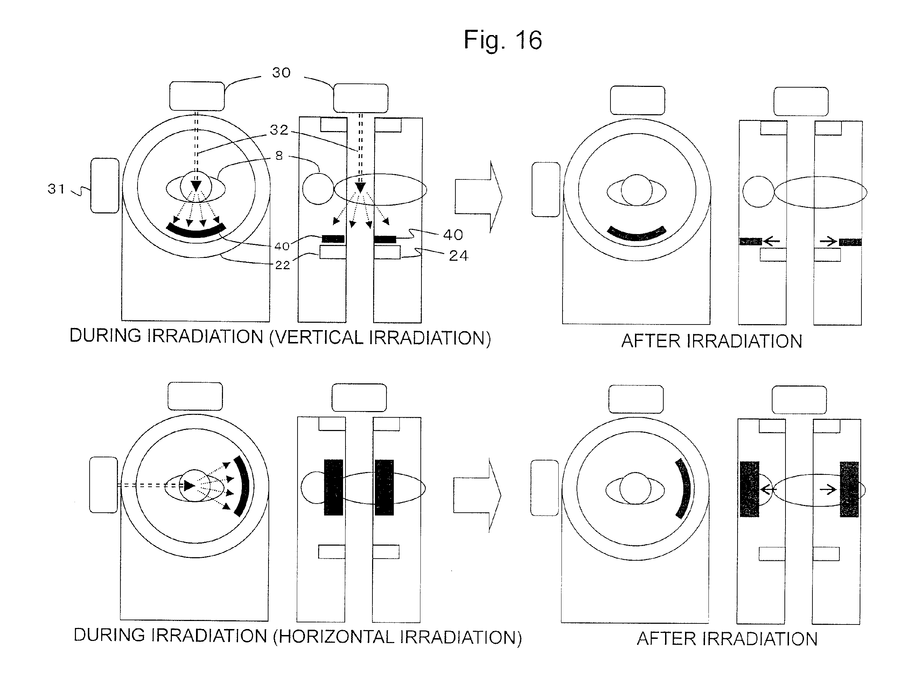

Specific examples will be described below. Figs. 15 and 16 show an example of shield retraction type with horizontal and vertical, two irradiation ports 30 and 31. Shields 40 have different thicknesses depending on the scattering angle of nuclear fragments 34. The detector rings each include a shield 40 which is mounted on a rotation direction rail 70. The diagrams show the shield positions for vertical irradiation. For horizontal irradiation, the shields are rotated by 90°. The shields 40 also have a mechanism of sliding in the Z-direction along with the rotation direction rails 70. The shields 40 are thereby retracted in response to the ON to OFF of irradiation. In the diagrams, 72 designates a rail guide, 74 designates a motor, 76 designates a wire, 78 designates pulleys, 80 designates rails installed on the floor of the therapy room, and 82 designates a carriage. The shield rotating mechanism is not needed if there is only one fixed irradiation port.

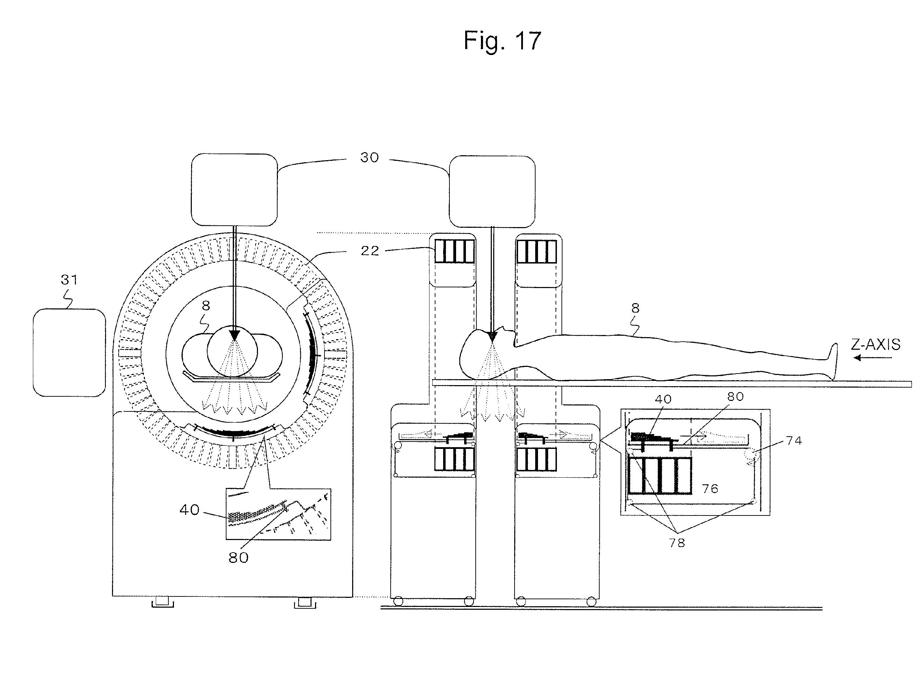

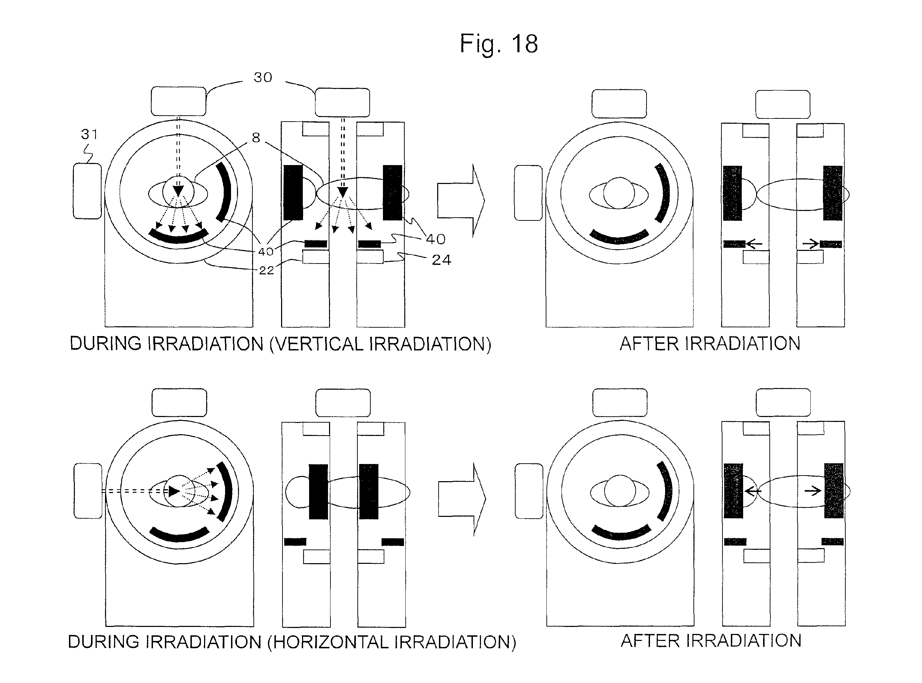

Figs. 17 and 18 show another example of shield retraction type with the same horizontal and vertical, two irradiation ports 30 and 31. Each detector ring includes two shields 40 to constitute a simplified configuration where the mechanism for rotating the shields 40 is omitted. In the diagrams, 84 designates Z-axis direction rails. Even in such a configuration, the detector rings may each include a single shield if there is only one fixed irradiation port.

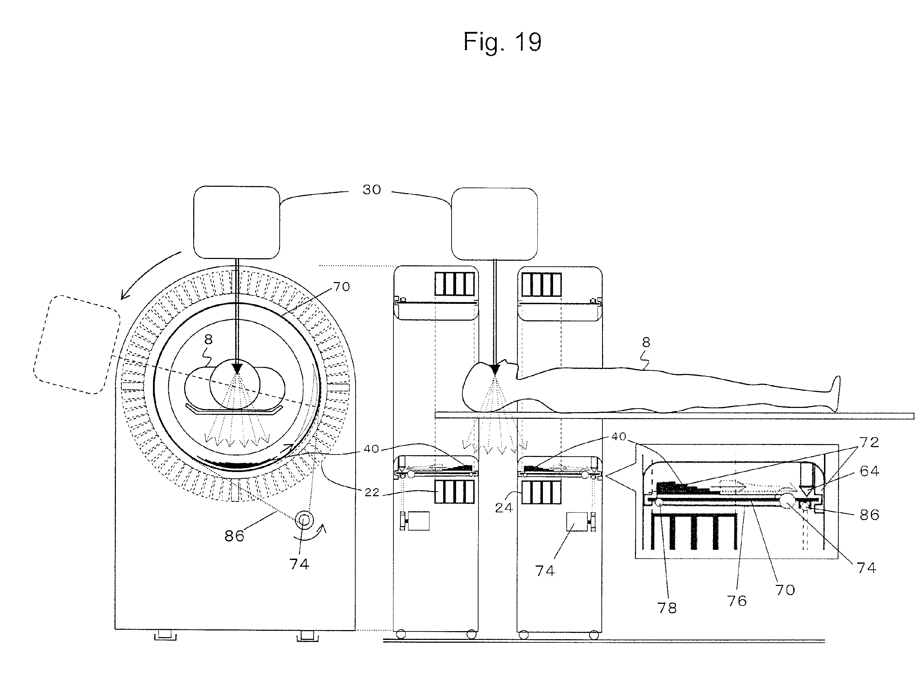

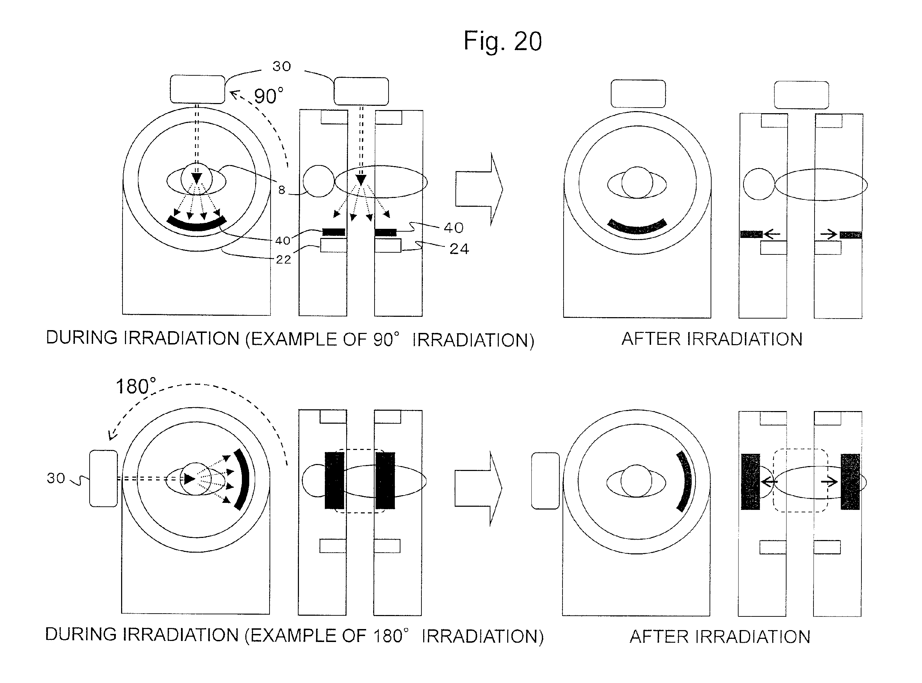

Figs. 19 and 20 show a configuration corresponding to a rotating irradiation gantry. The detector rings include a shield 40 each. The shields 40 are capable of a 360° rotation on rotation rails 70. The shields 40 also slide in the Z-direction within the rotation rails 70. The shields 40 are moved to an opposing side according to the movement of the irradiation port 30. The shields 40 are retracted in response to the ON to OFF of irradiation. In the diagram, 86 designates a belt.

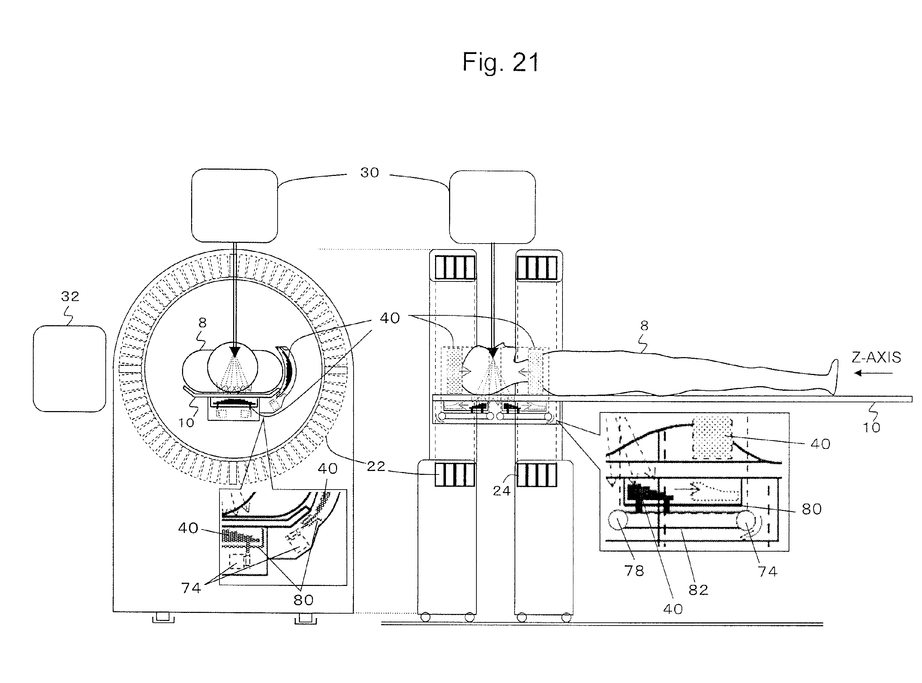

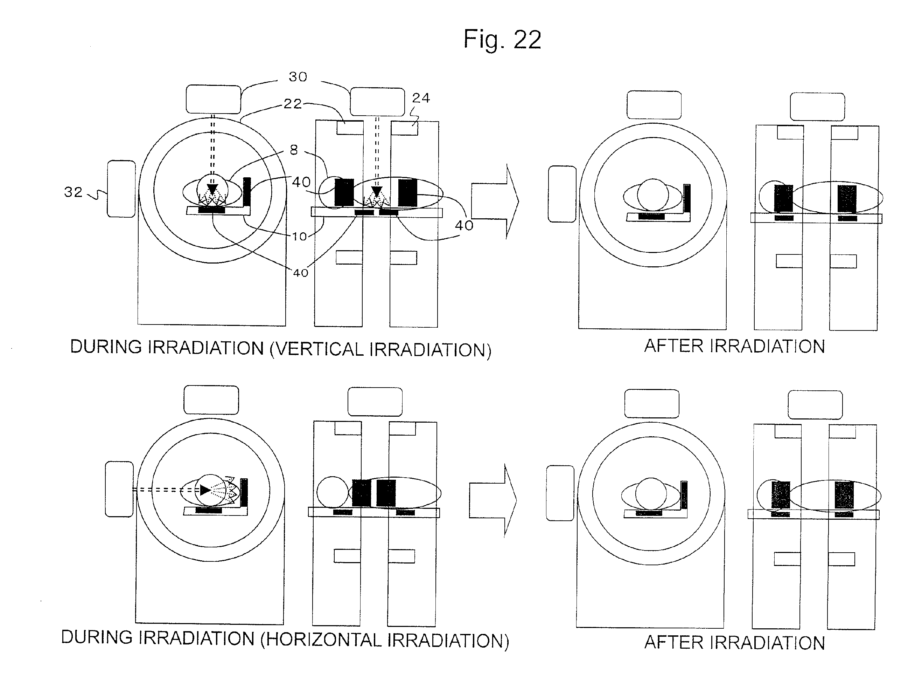

Figs. 21 and 22 show a configuration where shields 40 are built in a bed 10 instead of the gantries. Such a configuration has the advantage that the shields can be reduced in size and weight since the shields can be installed near the irradiation field. There are shields 40 for vertical irradiation and horizontal irradiation separately, both having a mechanism of sliding in the Z direction. Even in such a configuration, the shields may be reduced in number if there is only one fixed irradiation port.

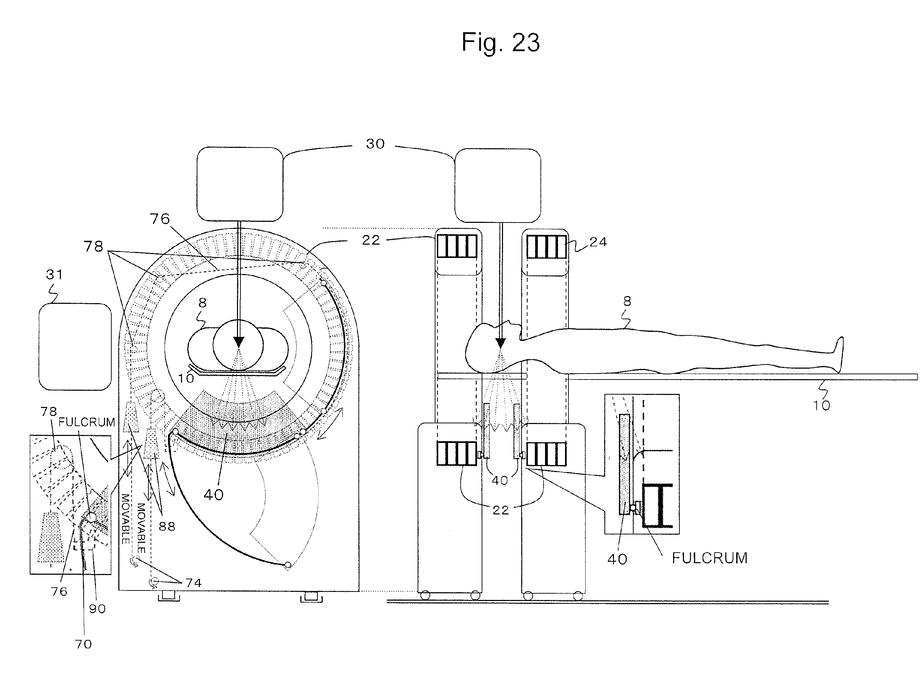

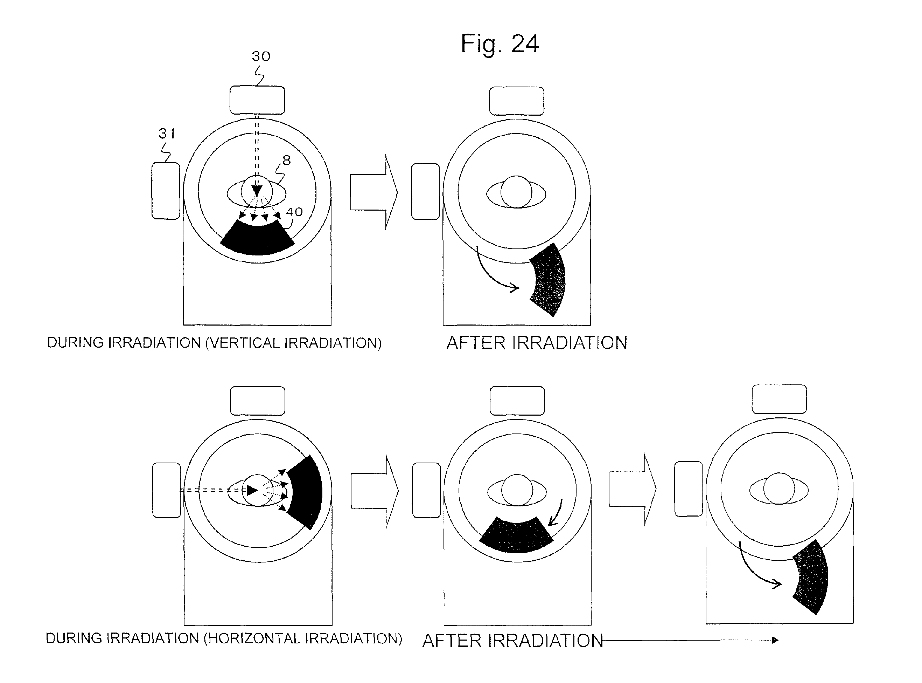

Figs. 23 and 24 show a configuration in which shields are not arranged in parallel with the Z-axis. Instead, arc-shaped end shields 41 are installed as shields. Fig. 23 shows the positions of the shields 41 for vertical irradiation. The shields 41 are configured to be rotatable in the directions of the arrow B. For horizontal irradiation, the shields 41 are moved to a position 41A opposite to the irradiation port 31. In such an example, complicated mechanisms need not be installed in front of the detectors. This improves the detection sensitivity of annihilation radiations and allows designing a wide patient port. Since the shields are retracted along rails 87 in the direction of gravitational force, it is possible to remove the shields promptly after irradiation. In the diagram, 88 designates weights, and 90 designates a hook.

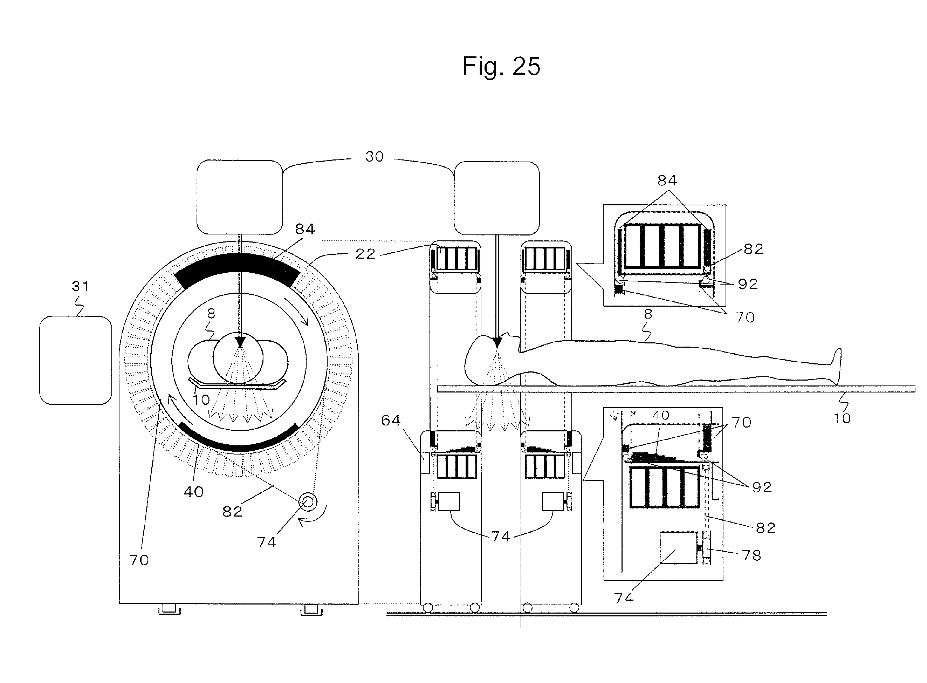

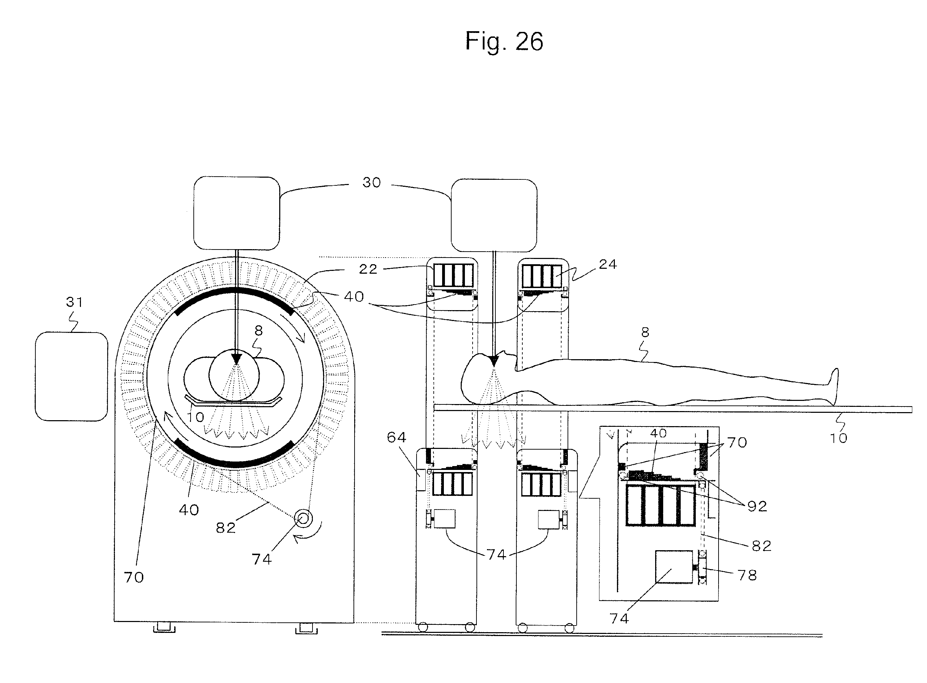

Figs. 25 and 26 show the configurations of examples of shield rotation type where each detector ring includes one shield 40 (Fig. 25) and two shields 40 (Fig. 26), respectively. In the example of Fig. 25, arch-shaped weights 88 are arranged in positions opposite to the shields 40. In the diagrams, 92 designate ball bearings.

Industrial Applicability

When performing monitoring for detecting annihilation radiations occurring from an irradiation field due to radiation irradiation in radiation therapy which is conducted by irradiating an affected area with X-rays or a particle beam, the incidence of nuclear fragments accompanying the beam irradiation on detectors can be reduced to enable measurement of annihilation radiations and three-dimensional imaging of the irradiation field immediately after irradiation or even during irradiation.

**Claims**

1. A shield type radiation therapy and imaging hybrid device including an imaging device that has a detector arranged so as to be able to measure a secondary radiation occurring from an affected area due to radiation irradiation and images an irradiation field after irradiation or during irradiation in synchronization with a radiation with which a field of view of the detector is irradiated,
   the hybrid device comprising:

   a radiation therapy device that irradiates a part of a subject with a radiation, the part lying in a field of view of the imaging device; and
   shielding means for lessening incidence of nuclear fragments on the detector, the nuclear fragments flying forward in a direction of irradiation from the subject due to the radiation irradiation, the shielding means being arranged in a flying space of the nuclear fragments.

2. A shield type radiation therapy and imaging hybrid device including a PET device that has a group of detectors arranged around a subject so as to be capable of coincidence measurement of a pair of annihilation radiations occurring from the subject and performs tomographic scanning on the subject after irradiation or during irradiation in synchronization with a radiation with which a field of view of the group of detectors is irradiated,
   the hybrid device comprising:

   a radiation therapy device that irradiates a part of the subject with a radiation, the part lying in a field of view of the PET device; and
   shielding means for lessening incidence of nuclear fragments on the detectors, the nuclear fragments flying forward in a direction of irradiation from the subject due to the radiation irradiation, the shielding means being arranged in a flying space of the nuclear fragments.

3.  The shield type radiation therapy and imaging hybrid device according to claim 2, wherein the group of detectors is arranged in a ring shape around an axis of the subject; the shielding means includes a shield that can be inserted and retracted into/from the flying space and lessens passage of nuclear fragments, and control means for controlling the insertion and retraction of the shield; and the shield is located in the flying space during radiation irradiation to lessen incidence of fragments into the detectors.

4.  The shield type radiation therapy and imaging hybrid device according to claim 3, wherein the shield makes a rotational movement.

5.  The shield type radiation therapy and imaging hybrid device according to claim 3, wherein the shield makes a reciprocal movement.

6.  The shield type radiation therapy and imaging hybrid device according to claim 3, wherein the shield is immediately retracted after radiation irradiation.

7.  The shield type radiation therapy and imaging hybrid device according to claim 3, wherein the shield is rotated depending on a position of an irradiation port.

8.  The shield type radiation therapy and imaging hybrid device according to claim 3, wherein the shield and a drive unit thereof are integrated with a bed.

9.  The shield type radiation therapy and imaging hybrid device according to claim 3, wherein a material or thickness of the shield is changed according to a scattering angle of the nuclear fragments.

10. A control program of a detector rotation type radiation therapy and imaging hybrid device including an imaging device that has a detector arranged so as to be able to measure a radiation occurring from an affected area due to radiation irradiation and images an irradiation field after irradiation or during irradiation in synchronization with a radiation with which a field of view of the detector is irradiated,
    the control program controlling shielding means so as to lessen incidence of nuclear fragments on the detector, the nuclear fragments flying forward in a direction of irradiation from the subject due to the radiation irradiation, the shielding means being arranged in a flying space of the nuclear fragments.

11. A control program of a detector rotation type radiation therapy and imaging hybrid device including a PET device that has a group of detectors arranged around a subject so as to be capable of coincidence measurement of a pair of annihilation radiations occurring from the subject and performs tomographic scanning on the subject after irradiation or during irradiation in synchronization with a radiation with which a field of view of the group of detectors is irradiated,
    the control program controlling shielding means so as to lessen incidence of nuclear fragments on the detectors, the nuclear fragments flying forward in a direction of irradiation from the subject due to the radiation irradiation, the shielding means being arranged in a flying space of the nuclear fragments.

Fig. 1

# Fig. 2

IRRADIATION FIELD

30

32

8

R

θc

20

34

Wc

## Fig. 3

DURING IRRADIATION

AFTER IRRADIATION

Fig. 4

# Fig. 5

```
                    ( START )
                        |
                        v
  100 —    IRRADIATION PREPARATION
               INSTRUCTION
                        |
                        v
  102 —      SHIELD INSTALLATION
                 INSTRUCTION
                        |
                        v
  104       IS SHIELD INSTALLATION
                COMPLETED?
                        |
                        v
  106 —        START IRRADIATION
                        |
                        v
  108 —         END IRRADIATION
                        |
                        v
  110 —         RETRACT SHIELDS
                        |
                        v
  112              END
               TREATMENT?
                        |
                        v
                    ( END )
```

## Fig. 6

IRRADIATION | ON | OFF | ON | OFF

SHIELD | IN PLACE | RETRACTED | IN PLACE | RETRACTED

CONTINUOUS MEASUREMENT

PET MEASUREMENT (PARTIAL DATA) (COMPLETE DATA) (PARTIAL DATA) (COMPLETE DATA)

IMAGE RECONSTRUCTION

IMAGING IS POSSIBLE IN ARBITRARY TIME FRAME

EP 2 412 404 A1

# Fig. 7

ORBIT OF
SHIELD ROTATION

θd

θc

θs

PET
FIELD OF VIEW

Wc

30

32

40

40

# Fig. 8

EP 2 412 404 A1

- 54
- 52
- SYNCHRONOUS SIGNAL
- 60
- ACCELERATOR CONTROL SYSTEM
- SHIELD CONTROL SYSTEM
- SHIELD ROTATION CONTROL SIGNAL
- 20
- 42
- SHIELD CONTROL INFORMATION
- 32
- 42
- 30
- 8
- 62
- MOTOR CONTROL DEVICE
- 44
- COINCIDENCE CIRCUIT
- ROTATION SENSOR
- 46
- DATA COLLECTION SYSTEM
- 64
- SHIELD CONTROL INFORMATION
- 48
- IMAGE RECONSTRUCTION SYSTEM
- 50
- DISPLAY AND STORAGE SYSTEM

18

# Fig. 9

```
                    ┌──────────────────┐
                    │      START       │
                    └──────────────────┘
                              │
                              ▼
100 ──┐   ┌────────────────────────────────────────────┐
      └─  │   IRRADIATION PREPARATION INSTRUCTION        │
          └────────────────────────────────────────────┘
                              │
                              ▼ ◄──────────────────────┐
202 ──┐   ┌─────────────────────────────┐              │
      └─  │     ADJUST SHIELD ROTATION   │              │
          └─────────────────────────────┘              │
                              │                         │
                              ▼                         │
204 ──┐           ╱────────────────────────╲           │
      └─         ╱   ARE IRRADIATION AND     ╲          │
                ╱   ROTATION SYNCHRONIZED?    ╲─── N ───┘
                ╲                             ╱
                 ╲───────────────────────────╱
                              │ Y
                              ▼ ◄──────────────────────┐
206 ──┐   ┌─────────────────────────────────────┐      │
      └─  │   ROTATION SYNCHRONOUS IRRADIATION   │      │
          └─────────────────────────────────────┘      │
                              │                         │
                              ▼                         │
112 ──┐           ╱────────────────────╲                │
      └─         ╱    END TREATMENT?     ╲──── N ────────┘
                 ╲                       ╱
                  ╲─────────────────────╱
                              │ Y
                              ▼
                    ┌──────────────────┐
                    │       END        │
                    └──────────────────┘
```

# Fig. 10

```
                    ┌─────────────┐
                    │   START     │
                    └──────┬──────┘
                           │
100 ──── ┌─────────────────▼────────────────────┐
         │ IRRADIATION PREPARATION INSTRUCTION   │
         └─────────────────┬────────────────────┘
                           │
                           ▼◄──────────────────────┐
202 ──── ┌────────────────────────────┐            │
         │   ADJUST SHIELD ROTATION    │            │
         └─────────────┬──────────────┘            │
                       │                            │
204 ──────            ◄▼►                           │
              ARE IRRADIATION AND ROTATION ─────────┘
                    SYNCHRONIZED?          N
                       │
                       Y
                       ▼◄──────────────────────────┐
                       ▼◄─────────────────────────┐│
208 ──────            ◄▼►                          ││
                 IRRADIATION TIMING? ──────────────┘│
                       │              N             │
                       Y                            │
210 ──── ┌────────────────────────┐                │
         │      IRRADIATION        │                │
         └────────────┬───────────┘                │
                      │                             │
112 ──────           ◄▼►                            │
                END TREATMENT? ────────────────────┘
                      │            N
                      Y
                      ▼
                ┌───────────┐
                │    END    │
                └───────────┘
```

## Fig. 11

IMAGING IS POSSIBLE IN ARBITRARY TIME FRAME OF T(=ti+ts) OR LONGER

# Fig. 12

ORBIT OF
SHIELD ROTATION

PET
FIELD OF VIEW

$2\cos^{-1}(r/R)$

# Fig. 13

30

20

8

42

θc

θc

IRRADIATION
SYSTEM

| IRRADIATION | | TIME (SEC) |

ti

ts

SHIELD
ROTATION

0

360° θ

θs−θc
(=180° −θd−θc)

ANGULAR SPEED: 360° ／T

# Fig. 14

32

20

8

10

34

EP 2 412 404 A1

## Fig. 15

# Fig. 16

DURING IRRADIATION (VERTICAL IRRADIATION)

AFTER IRRADIATION

DURING IRRADIATION (HORIZONTAL IRRADIATION)

AFTER IRRADIATION

EP 2 412 404 A1

# Fig. 17

EP 2 412 404 A1

Fig. 18

DURING IRRADIATION (VERTICAL IRRADIATION)

AFTER IRRADIATION

DURING IRRADIATION (HORIZONTAL IRRADIATION)

AFTER IRRADIATION

Fig. 19

# Fig. 20

DURING IRRADIATION (EXAMPLE OF 90° IRRADIATION)    AFTER IRRADIATION

DURING IRRADIATION (EXAMPLE OF 180° IRRADIATION)    AFTER IRRADIATION

EP 2 412 404 A1

Fig. 21

EP 2 412 404 A1

Fig. 22

DURING IRRADIATION (VERTICAL IRRADIATION)

AFTER IRRADIATION

DURING IRRADIATION (HORIZONTAL IRRADIATION)

AFTER IRRADIATION

EP 2 412 404 A1

Fig. 23

# Fig. 24

DURING IRRADIATION (VERTICAL IRRADIATION)    AFTER IRRADIATION

DURING IRRADIATION (HORIZONTAL IRRADIATION)    AFTER IRRADIATION

EP 2 412 404 A1

Fig. 25

EP 2 412 404 A1

# Fig. 26

<div align="center">

**INTERNATIONAL SEARCH REPORT**

</div>

| | International application No. |
|---|---|
| | PCT/JP2009/055702 |

A. CLASSIFICATION OF SUBJECT MATTER
*A61N5/10*(2006.01)i

According to International Patent Classification (IPC) or to both national classification and IPC

B. FIELDS SEARCHED

Minimum documentation searched (classification system followed by classification symbols)
A61N5/10, G21F1/08

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus(JDreamII), JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2008/129666 A1 (National Institute of Radiological Sciences),<br>30 October, 2008 (30.10.08),<br>Par. Nos. [0041] to [0086]; Fig. 25<br>(Family: none) | 1-5,7-11<br>6 |
| Y | JP 2006-329915 A (Akita University et al.),<br>07 December, 2006 (07.12.06),<br>Par. No. [0005]<br>(Family: none) | 1-5,7-11 |
| A | JP 2008-173299 A (President of National Cancer Center et al.),<br>31 July, 2008 (31.07.08),<br>Full text; all drawings<br>(Family: none) | 1-11 |

| ☒ Further documents are listed in the continuation of Box C. | ☐ See patent family annex. |
|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 April, 2009 (30.04.09) | 19 May, 2009 (19.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/055702

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2008-022994 A  (Japan Atomic Energy Agency), 07 February, 2008 (07.02.08), Full text; all drawings (Family: none) | 1-11 |
| A | JP 09-189769 A  (Mitsubishi Electric Corp.), 22 July, 1997 (22.07.97), Full text; all drawings (Family: none) | 1-11 |

Form PCT/ISA/210 (continuation of second sheet) (April 2007)

## REFERENCES CITED IN THE DESCRIPTION

**Patent documents cited in the description**

- JP 2008022994 A

- JP 2008173299 A

**Non-patent literature cited in the description**

- **W. Enghardt et al.** Charged hadron tumour therapy monitoring by means of PET. *Nucl. Instrum. Methods A,* 2004, vol. 525, 284-288
- **S. Janek et al.** Development of dose delivery verification by PET imaging of photonuclear reactions following high energy photon therapy. *Phys. Med. Biol.,* 2006, vol. 51, 5769-5783
- **N. Matsufuji et al.** Spatial fragmentation distribution from a therapeutic pencil-like carbon beam in water. *Physics in Medicine and Biology,* 2005, vol. 50, 3393-3403
- **S. Yonai et al.** Measurement of neutron ambient dose equivalent in passive carbon-ion and proton radiotherapies. *Medical Physics,* 2008, vol. 35, 4782-4792

- **P. Crespo et al.** On the detector arrangement for in-beam PET for hadron therapy monitoring. *Phys. Med. Biol.,* 2006, vol. 51, 2143-2163
- **T. Nishio et al.** Dose-volume delivery guided proton therapy using beam ON-LINE PET system. *Med. Phys.,* 2006, vol. 33, 4190-4197
- **Taiga Yamaya ; Taku Inaniwa ; Shinichi Minohara ; Eiji Yoshida ; Naoko Inadama ; Fumihiko Nishikido ; Kengo Shibuya ; Chih Fung Lam ; Hideo Murayama.** A proposal of an open PET geometry. *Phy. Med. Biol.,* 2008, vol. 53, 757-773
- **P. Crespo et al.** Suppression of random coincidences during in-beam PET measurements at ion beam radiation therapy facilities. *IEEE TRANSACTIONS ON NUCLEAR SCIENCE,* August 2005, vol. 52 (4